# EUROPEAN PATENT SPECIFICATION

(11) **EP 3 601 611 B1**
(45) Date of publication and mention of the grant of the patent: **05.01.2022**
(21) Application number: 18717475.0
(22) Date of filing: 22.03.2018
(51) Int. Cl.: C12Q 1/6855

(54) **POLYNUCLEOTIDE ADAPTERS AND METHODS OF USE THEREOF**
POLYNUKLEOTIDADAPTER UND VERFAHREN ZUR VERWENDUNG DAVON
ADAPTATEURS POLYNUCLÉOTIDIQUES ET PROCÉDÉS D'UTILISATION DE CES DERNIERS

(30) Priority: 24.03.2017 US 201762476541 P
(43) Date of publication of application: 05.02.2020
(73) Proprietor: Life Technologies Corporation, Carlsbad, CA 92008 (US)
(72) Inventor: ANDERSEN, Mark, Carlsbad California 92008 (US); ALLEN, Michael, Carlsbad California 92008 (US)
(74) Representative: Thermo Fisher Scientific- Life Sciences Solutions Group
(86) International application number: PCT/US2018/023872
(87) International publication number: WO 2018/175798

(56) References cited:
- WO-A1-2012/033687
- US-A1- 2012 108 440
- MITSUOKI KAWANO ET AL: "Reduction of non-insert sequence reads by dimer eliminator LNA oligonucleotide for small RNA deep sequencing", BIOTECHNIQUES, INFORMA HEALTHCARE, UNITED STATES, vol. 49, no. 4, 1 October 2010 (2010-10-01), pages 751-755, XP002667963, ISSN: 1940-9818, DOI: 10.2144/000113516
- SABRINA SHORE ET AL: "Small RNA Library Preparation Method for Next-Generation Sequencing Using Chemical Modifications to Prevent Adapter Dimer Formation", PLOS ONE, vol. 11, no. 11, 22 November 2016 (2016-11-22), page e0167009, XP055476686, DOI: 10.1371/journal.pone.0167009
- (TM) Shore ET AL: "The Modified Nucleic Acid Experts Pushing the Limits of Small RNA-Seq with Chemically Modified Adapters", , 10 November 2014 (2014-11-10), XP55476664, Retrieved from the Internet: URL:http://www.trilinkbiotech.com/work/sRN APrep.pdf [retrieved on 2018-05-18]
- PING XU ET AL: "An improved protocol for small RNA library construction using High Definition adapters", METHODS IN NEXT GENERATION SEQUENCING, vol. 2, no. 1, 1 January 2015 (2015-01-01) , pages 1-10, XP055462688, DOI: 10.1515/mngs-2015-0001

## Description

This application claims priority to and the benefit of U.S. Provisional Application No. 62/476,541, filed March 24, 2017.

Throughout this application various publications, patents, and/or patent applications are referenced. The disclosures of the publications, patents and/or patent applications are provided by this application in order to more fully describe the state of the art to which this invention pertains.

### FIELD OF THE INVENTION

The present invention relates to novel polynucleotide adapters for use in library preparation and sequencing methods.

### SUMMARY OF THE INVENTION

Improved methods of creating libraries of nucleic acid molecules for analysis (e.g., sequencing) have been developed. Provided are methods and compositions for reducing unfavorable dimer formation and thereby improving library preparation, e.g., for sequencing. Methods include addition of adapters (i.e., sequences) to ends of target nucleic acid(s) to facilitate amplification and analysis of such sequences.

For example, adapters that contain primer sequences can be ligated onto the ends of target nucleic acid sequences. A single adapter or two different adapters can be used in the ligation reaction. Such methods enable multiple target nucleic acid molecules of the same or different, known or unknown sequence to be amplified in a single amplification reaction. Such target molecules can then be used in, for example, analysis methods such as, e.g., sequencing techniques. One drawback of preparing routine libraries includes the formation of adapter-dimers, is reduced in adoption methods comprising use of provided compositions herein.

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

### DESCRIPTION OF THE DRAWINGS

FIGURE 1A depicts exemplary adapters provided and used in the methods herein. Phosphorothioate linkages are denoted by an asterisk.
FIGURE 1B depicts exemplary adapters provided and used in the methods herein.
FIGURE 1C depicts exemplary adapters provided and used in the methods herein.
FIGURE 1D depicts exemplary adapters provided and used in the methods herein. Phosphorothioate linkages are denoted by an asterisk.
FIGURE 2 is a graph depicting library yield, as measured by qPCR. Full length (Std), 3' modified full length adapter (AA), unprotected short (34), amino protected short (34Amino), and phophorothioate protected short (34 pT) adapters were used in amplicon ligation reactions in duplicate.
FIGURE 3 depicts sequencing performance of adapters, as determined by uniformity, end to end sequencing, and strand bias. Full length (Std), 3' modified full length adapter (AA), unprotected short (34), amino protected short (34Amino), and phophorothioate protected short (34 pT) adapters were each ligated to amplicon libraries, followed by sequence performance evaluation.

### DETAILED DESCRIPTION OF THE INVENTION

Adapter dimer is problematic in NGS applications because it can be efficiently amplified and will bind to the flow cell in a sequencing reaction, however, will produce useless data. Because of this, it is important that adapters produce as little dimers as possible. MITSUOKI KAWANO ET AL, "Reduction of non-insert sequence reads by dimer eliminator LNA oligonucleotide for small RNA deep sequencing", BIOTECHNIQUES, vol. 49, no. 4, 2010, pages 751 - 755 discloses a method to reduce adapter-dimers that capture and eliminate dimers by addition of a separate 'dimer eliminator' construct. The construct prevents reverse transcription of the dimers using a locked nucleic acid (LNA) oligonucleotide-named dimer eliminator-that is complementary to the adapter-dimer ligation products during the reverse transcription reaction. PING XU ET AL, "An improved protocol for small RNA library construction using High Definition adapters", METHODS IN NEXT GENERATION SEQUENCING, vol. 2, no. 1, 2015, pages 1 - 10 discloses a protocol to generate sRNA libraries using HD adapters with greatly reduced proportion of adapter-adapter products due to the degradation of nonligated 3' adapters. WO2012033687 discloses a method wherein the adaptors are added sequentially and a blocking oligonucleotide is added to hybridise to excess adapter and prevent it from forming dimers with the second adaptor. US2012108440 provides a method of reducing adapter dimer formation comprising contacting a sample comprising target nucleic acid sequences with 5' and 3' adapters in the presence of one or more hairpin oligonucleotides under conditions to form 5'-adapter-target-3'-adapter sequences. Instead of adding separate molecules to reduce dimer formation SABRINA SHORE ET AL, "Small RNA Library Preparation Method for Next-Generation Sequencing Using Chemical Modifications to Prevent Adapter Dimer Formation", PLOS ONE, 2016, vol. 11, no. 11, page e0167009 and Shore ET AL, "The Modified Nucleic Acid Experts Pushing the Limits of Small RNA-Seq with Chemically Modified Adapters", 2014, URL: http://www.trilinkbiotech.com/work/sRNAPrep.pdf, disclose a library preparation approach that uses modified adapters to suppress adapter dimer formation. The method employs chemically modified adapters to prevent adapter dimer formation by blocking ligation of the 5' and 3' adapters to one another, while allowing for efficient tagging of adapters onto a small RNA library. Since the the chemical modifications are thought to prevent reverse transcriptase (RT) read-through during the cDNA synthesis step the method is applicable to RNA library construction only and cannot be applied when generating libraries from DNA. Thus, provided are improved compositions and methods for reduction of dimer formation and improved library preparation.

In one aspect, provided are compositions comprising an adapter sequence having a 5' extended overhang sequence and a 3' blunt end or T overhang sequence, wherein the adapter comprises short reverse complementary sequences over less than 60, 55, 50, 45, or 40 percent of the length at its 3' end. The 5' adapter sequence and 3' adapter sequence are capable of ligating to amplicon target sequences of interest.

In some embodiments, the adapter comprises a nucleic acid, including DNA, RNA, RNA/DNA molecules, or analogs thereof. In some embodiments, the adapter can include one or more deoxyribonucleoside or ribonucleoside residues. In some embodiments, the adapter can be single-stranded or double-stranded nucleic acids, or can include single-stranded and/or double-stranded portions.

In some embodiments, the adapter can have any length, including fewer than 10 bases in length, or about 10-20 bases in length, or about 20-50 bases in length, or about 25-75 bases in length, or longer.

In some embodiments, the adapter can include a nucleotide sequence that is identical or complementary to any portion of the target polynucleotide, capture primer, fusion primer, solution-phase primer, amplification primer, or a sequencing primer.

In some embodiments, the adapter can have a 5' overhang tail. In some embodiments, the tail can be any length, including 1-50 or more nucleotides in length.

In some embodiment, the 5' and 3' adapters each comprise short reverse complementary sequences over less than 65, 60, 55, 50, 45, or 40 percent of the length at its 3' end. In some embodiments the 5' adapters comprise short reverse complementary sequences over less than 60, 55, 50, 45, or 40 percent of the length at its 3' end. In other embodiments the 3' adapters comprise short reverse complementary sequences over less than 60, 55, 50, 45, or 40 percent of the length at its 3' end.

In some embodiments, the adapters comprise single stranded 5' overhang. In particular embodiments, the 5' adapters comprise single stranded 5' overhang. In additional or other particular embodiments, the 3' adapters comprise single stranded 5' overhang. In certain embodiments, the adapters comprise 5' overhang sequences over at least 30, 40, 45, 50, 55, or 60 percent of the length.

In some embodiments, the adapters comprise modified sequences. In particular embodiments, an adapter is a 3'-phosphorothioate protected adapter. In another particular embodiment(s), an adapter is a 3'-amino modified adapter.

In some embodiments, the adapter can include degenerate sequences. In some embodiments, the adapter can include one or more inosine residues. In some embodiments, the adapter can include at least one scissile linkage. In some embodiments, the scissile linkage can be susceptible to cleavage or degradation by an enzyme or chemical compound. Optionally, the adapter includes at least one uracil base. In some embodiments, the adapter can include at least one phosphorothiolate, phosphorothioate, and/or phosphoramidate linkage.

In some embodiments, the adapter can have any combination of blunt end(s) and/or sticky end(s). In some embodiments, at least one end of the adapter can be compatible with at least one end of a nucleic acid fragment. In some embodiments, a compatible end of the adapter can be joined to a compatible end of a nucleic acid fragment. In some embodiments, the adapter can have a 5' or 3' overhang end.

In some embodiments, the 5' and 3' adapters are linear. In some embodiments, the 5' and 3' adapters are blunt ended. In some embodiments, the 5' and 3' adapters comprise T overhangs.at their 3' end. In certain embodiments one of the 5' and 3' adapters are blunt ended and one of the 5' and 3' adapters comprise T overhangs.at their 3' end.

In some embodiments, the reverse complement oligonucleotide adapter sequence is selected from the group consisting of SEQ ID NO:3, 4, or 5 or selected from the group consisting of SEQ ID NO:7, 8, or 9.

In particular embodiments, a forward oligonucleotide adapter sequence comprises SEQ ID NO:1 and a reverse complement oligonucleotide adapter sequence is selected from the group consisting of SEQ ID NO:3, 4, or 5. In particular embodiments, a forward oligonucleotide adapter sequence comprises SEQ ID NO:6 and a reverse complement oligonucleotide adapter sequence is selected from the group consisting of SEQ ID NO:7, 8, or 9.

In certain embodiments, a composition comprises each of a 5' adapter and a 3' adapter oligonucleotide.

In some embodiments the first and second adapters have universal sequences.

In some embodiments, the adapter can include a unique identifier sequence (e.g., barcode or index sequence). In some embodiments, a barcoded adapter can be used for constructing a multiplex library of target polynucleotides. In some embodiments, the barcoded adapters can be appended to a target polynucleotide and used for sorting or tracking the source of the target polynucleotide. In some embodiments, one or more barcode/index sequences can allow identification of a particular adapter among a mixture of different adapters having different barcodes sequences. For example, a mixture can include 2, 3, 4, 5, 6, 7-10, 10-50, 50-100, 100-200, 200-500, 500-1000, or more different adapters having unique barcode sequences.

In some embodiments, the adapter can include any type of restriction enzyme recognition sequence, including type I, type II, type lIs, type IIB, type III, type IV restriction enzyme recognition sequences, or recognition sequences having palindromic or non-palindromic recognition sequences.

In some embodiments, the adapter can include a cell regulation sequences, including a promoter (inducible or constitutive), enhancers, transcription or translation initiation sequence, transcription or translation termination sequence, secretion signals, Kozak sequence, cellular protein binding sequence, and the like.

In another aspect, provided are methods of reducing adapter dimer formation. In certain embodiments the method comprises contacting a sample comprising target nucleic acid sequences of interest with 5 ' and 3 ' adapters of the invention under conditions to form 5'-adapter-target-3'-adapter sequences. In some embodiments, the 5' and 3' adapters each comprise short reverse complementary sequences over less than seventy percent (70%) of the adapter length at its 3' end. In such methods the amount of adapter dimer formation is reduced compared to the amount in the absence of the oligonucleotides (e.g., in the presence of adapter sequences having full length reverse complementary sequences). In some embodiments, less than 25, 20, 15, 10, 8, 6, 5, 4, 3, 2, or 1% of adapters form dimers resulting from the method.

In some embodiments, the method comprises use of 5' and 3' adapters each comprising short reverse complementary sequences over less than 65, 60, 55, 50, 45, or 40 percent of the length at its 3' end. In particular embodiments, the 5' adapters comprise short reverse complementary sequences over less than 60, 55, 50, 45, or 40 percent of the length at its 3' end. In additional or other particular embodiments, the 3' adapters comprise short reverse complementary sequences over less than 60, 55, 50, 45, or 40 percent of the length at its 3' end.

In another aspect, provided are methods of preparing a library of nucleic acid sequences. In certain embodiments the method comprises contacting first and second adapter oligonucleotides of the invention with a sample comprising target nucleic acid sequences under conditions to form to form 5'-adapter-target-3'-adapter ligation products. In some embodiments; adapter oligonucleotides comprise 5 ' and 3 ' adapters each having short reverse complementary sequences over less than seventy percent (70%) of the adapter length at its 3' end and form ligation products, wherein the ligation products form the library of nucleic acid sequences. In such methods the amount of adapter dimer formation is reduced compared to the amount in the absence of the oligonucleotides (e.g., in the presence of adapter sequences having full length reverse complementary sequences). In some embodiments, less than 25, 20, 15, 10, 8, 6, 5, 4, 3, 2, or 1% of adapters form dimers resulting from the method.

In some embodiments, provided methods further comprising amplifying the ligation products to product the library of nucleic acid sequences. In some additional embodiments, provided methods further comprise analysis of the sequence of the ligation products. In particular embodiments, the analysis method comprises sequencing the ligation products.

In some embodiments the oligonucleotide adapters are not complementary to the target nucleic acid sequences of interest. Preferably, target nucleic acid molecules comprise two or more and up to 100,000 different sequences.

In another aspect, provided are kits for reducing adapter formation and improved methods of preparing a library of nucleic acid sequences, comprising one or more of the provided adapter compositions herein. In some embodiments, kits comprise two or more adapters. In particular embodiments, kits comprise a 5' adapter and a 3' adapter for use in the methods provided herein. Optionally, kits comprise one or more components selected from buffers, enzymes (e.g., ligase, polymerase), dNTPs.

Provided compositions and components may be used in conjunction with additional compositions and methods described herein. Unless defined otherwise, all technical and scientific terms used herein have the same meaning as is commonly understood by one of ordinary skill in the art to which these inventions belong. If a definition and/or description is set forth herein that is contrary to or otherwise inconsistent with any definition set forth in the patents, patent applications, published applications, and other publications that are referenced herein, the definition and/or description set forth herein prevails over the definition that is disclosed in a referenced document.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

As used herein, the terms "adapter" or "adapter and its complements" and their derivatives, refers generally to any linear oligonucleotide of the disclosure which can be ligated to a target nucleic acid sequence. Optionally, the adapter includes a nucleic acid sequence that is not substantially complementary to the 3' end or the 5' end of at least one target sequences within the sample. In some embodiments, the adapter is substantially non-complementary to the 3' end or the 5' end of any target sequence present in the sample. In some embodiments, the adapter includes any single stranded or double-stranded linear oligonucleotide that is not substantially complementary to an amplified target sequence. In some embodiments, the adapter is substantially non-complementary to at least one, some or all of the nucleic acid molecules of the sample. In some embodiments, suitable adapter lengths are in the range of about 10-100 nucleotides, about 12-60 nucleotides and about 15-50 nucleotides in length. Generally, the adapter can include any combination of nucleotides and/or nucleic acids. In some aspects, the adapter can include one or more cleavable groups at one or more locations. In another aspect, the adapter can include a sequence that is substantially identical, or substantially complementary, to at least a portion of a primer, for example a universal primer. In some embodiments, the adapter can include a barcode or tag to assist with downstream cataloguing, identification or sequencing. In some embodiments, a single-stranded adapter can act as a substrate for amplification when ligated to an amplified target sequence, particularly in the presence of a polymerase and dNTPs under suitable temperature and pH.

As used herein, "amplify", "amplifying" or "amplification reaction" and their derivatives, refer generally to any action or process whereby at least a portion of a nucleic acid molecule (referred to as a template nucleic acid molecule) is replicated or copied into at least one additional nucleic acid molecule. The additional nucleic acid molecule optionally includes sequence that is substantially identical or substantially complementary to at least some portion of the template nucleic acid molecule. The template nucleic acid molecule can be single-stranded or double-stranded and the additional nucleic acid molecule can independently be single-stranded or double-stranded. In some embodiments, amplification includes a template-dependent in vitro enzyme-catalyzed reaction for the production of at least one copy of at least some portion of the nucleic acid molecule or the production of at least one copy of a nucleic acid sequence that is complementary to at least some portion of the nucleic acid molecule. Amplification optionally includes linear or exponential replication of a nucleic acid molecule. In some embodiments, such amplification is performed using isothermal conditions; in other embodiments, such amplification can include thermocycling. In some embodiments, the amplification is a multiplex amplification that includes the simultaneous amplification of a plurality of target sequences in a single amplification reaction. At least some of the target sequences can be situated on the same nucleic acid molecule or on different target nucleic acid molecules included in the single amplification reaction. In some embodiments, "amplification" includes amplification of at least some portion of DNA- and RNA-based nucleic acids alone, or in combination. The amplification reaction can include single or double-stranded nucleic acid substrates and can further including any of the amplification processes known to one of ordinary skill in the art. In some embodiments, the amplification reaction includes polymerase chain reaction (PCR).

As used herein, "amplification conditions" and its derivatives, generally refers to conditions suitable for amplifying one or more nucleic acid sequences. Such amplification can be linear or exponential. In some embodiments, the amplification conditions can include isothermal conditions or alternatively can include thermocyling conditions, or a combination of isothermal and themocycling conditions. In some embodiments, the conditions suitable for amplifying one or more nucleic acid sequences includes polymerase chain reaction (PCR) conditions. Typically, the amplification conditions refer to a reaction mixture that is sufficient to amplify nucleic acids such as one or more target sequences, or to amplify an amplified target sequence ligated to one or more adapters, e.g., an adapter-ligated amplified target sequence. Generally, the amplification conditions include a catalyst for amplification or for nucleic acid synthesis, for example a polymerase; a primer that possesses some degree of complementarity to the nucleic acid to be amplified; and nucleotides, such as deoxyribonucleotide triphosphates (dNTPs) to promote extension of the primer once hybridized to the nucleic acid. The amplification conditions can require hybridization or annealing of a primer to a nucleic acid, extension of the primer and a denaturing step in which the extended primer is separated from the nucleic acid sequence undergoing amplification. Typically, but not necessarily, amplification conditions can include thermocycling; in some embodiments, amplification conditions include a plurality of cycles where the steps of annealing, extending and separating are repeated. Typically, the amplification conditions include cations such as Mg++ or Mn++ (e.g., MgCl2, etc) and can also include various modifiers of ionic strength.

As used herein, "blunt-end ligation" and its derivatives, refers generally to ligation of two blunt-end double-stranded nucleic acid molecules to each other. A "blunt end" refers to an end of a double-stranded nucleic acid molecule wherein substantially all of the nucleotides in the end of one strand of the nucleic acid molecule are base paired with opposing nucleotides in the other strand of the same nucleic acid molecule. A nucleic acid molecule is not blunt ended if it has an end that includes a single-stranded portion greater than two nucleotides in length, referred to herein as an "overhang". In some embodiments, the end of nucleic acid molecule does not include any single stranded portion, such that every nucleotide in one strand of the end is based paired with opposing nucleotides in the other strand of the same nucleic acid molecule. In some embodiments, the ends of the two blunt ended nucleic acid molecules that become ligated to each other do not include any overlapping, shared or complementary sequence. Typically, blunted-end ligation excludes the use of additional oligonucleotide adapters to assist in the ligation of the double-stranded amplified target sequence to the double-stranded adapter, such as patch oligonucleotides as described in Mitra and Varley, US2010/0129874, published May 27, 2010. In some embodiments, blunt-ended ligation includes a nick translation reaction to seal a nick created during the ligation process.

As used herein, the terms "comprises," "comprising," "includes," "including," "has," "having" or any other variation thereof, are intended to cover a non-exclusive inclusion. For example, a process, method, article, or apparatus that comprises a list of features is not necessarily limited only to those features but may include other features not expressly listed or inherent to such process, method, article, or apparatus. Further, unless expressly stated to the contrary, "or" refers to an inclusive-or and not to an exclusive-or.

As used herein, "comparable maximal minimum melting temperatures" and its derivatives, refers generally to the melting temperature (Tm) of each nucleic acid fragment for a single adapter or target-specific primer after cleavage of the cleavable groups. The hybridization temperature of each nucleic acid fragment generated by a single adapter or target-specific primer is compared to determine the maximal minimum temperature required preventing hybridization of any nucleic acid fragment from the target-specific primer or adapter to the target sequence. Once the maximal hybridization temperature is known, it is possible to manipulate the adapter or target-specific primer, for example by moving the location of the cleavable group along the length of the primer, to achieve a comparable maximal minimum melting temperature with respect to each nucleic acid fragment.

The terms "complementary" and "complement" and their variants, as used herein, refer to any two or more nucleic acid sequences (e.g., portions or entireties of template nucleic acid molecules, target sequences and/or primers) that can undergo cumulative base pairing at two or more individual corresponding positions in antiparallel orientation, as in a hybridized duplex. Such base pairing can proceed according to any set of established rules, for example according to Watson-Crick base pairing rules or according to some other base pairing paradigm. Optionally there can be "complete" or "total" complementarity between a first and second nucleic acid sequence where each nucleotide in the first nucleic acid sequence can undergo a stabilizing base pairing interaction with a nucleotide in the corresponding antiparallel position on the second nucleic acid sequence. "Partial" complementarity describes nucleic acid sequences in which at least 20%, but less than 100%, of the residues of one nucleic acid sequence are complementary to residues in the other nucleic acid sequence. In some embodiments, at least 50%, but less than 100%, of the residues of one nucleic acid sequence are complementary to residues in the other nucleic acid sequence. In some embodiments, at least 70%, 80%, 90%, 95% or 98%, but less than 100%, of the residues of one nucleic acid sequence are complementary to residues in the other nucleic acid sequence. Sequences are said to be "substantially complementary" when at least 85% of the residues of one nucleic acid sequence are complementary to residues in the other nucleic acid sequence. In some embodiments, two complementary or substantially complementary sequences are capable of hybridizing to each other under standard or stringent hybridization conditions. "Non-complementary" describes nucleic acid sequences in which less than 20% of the residues of one nucleic acid sequence are complementary to residues in the other nucleic acid sequence. Sequences are said to be "substantially non-complementary" when less than 15% of the residues of one nucleic acid sequence are complementary to residues in the other nucleic acid sequence. In some embodiments, two non-complementary or substantially non-complementary sequences cannot hybridize to each other under standard or stringent hybridization conditions. A "mismatch" is present at any position in the two opposed nucleotides are not complementary. Complementary nucleotides include nucleotides that are efficiently incorporated by DNA polymerases opposite each other during DNA replication under physiological conditions. In a typical embodiment, complementary nucleotides can form base pairs with each other, such as the A-T/U and G-C base pairs formed through specific Watson-Crick type hydrogen bonding, or base pairs formed through some other type of base pairing paradigm, between the nucleobases of nucleotides and/or polynucleotides in positions antiparallel to each other. The complementarity of other artificial base pairs can be based on other types of hydrogen bonding and/or hydrophobicity of bases and/or shape complementarity between bases.

As used herein, "contacting" and its derivatives, when used in reference to two or more components, refers generally to any process whereby the approach, proximity, mixture or commingling of the referenced components is promoted or achieved without necessarily requiring physical contact of such components, and includes mixing of solutions containing any one or more of the referenced components with each other. The referenced components may be contacted in any particular order or combination and the particular order of recitation of components is not limiting. For example, "contacting A with B and C" encompasses embodiments where A is first contacted with B then C, as well as embodiments where C is contacted with A then B, as well as embodiments where a mixture of A and C is contacted with B, and the like. Furthermore, such contacting does not necessarily require that the end result of the contacting process be a mixture including all of the referenced components, as long as at some point during the contacting process all of the referenced components are simultaneously present or simultaneously included in the same mixture or solution. For example, "contacting A with B and C" can include embodiments wherein C is first contacted with A to form a first mixture, which first mixture is then contacted with B to form a second mixture, following which C is removed from the second mixture; optionally A can then also be removed, leaving only B. Where one or more of the referenced components to be contacted includes a plurality (e.g, "contacting a target sequence with a plurality of target-specific primers and a polymerase"), then each member of the plurality can be viewed as an individual component of the contacting process, such that the contacting can include contacting of any one or more members of the plurality with any other member of the plurality and/or with any other referenced component (e.g., some but not all of the plurality of target specific primers can be contacted with a target sequence, then a polymerase, and then with other members of the plurality of target-specific primers) in any order or combination.

As used herein, "DNA barcode" or "DNA tagging sequence" or "index" and its derivatives, refers generally to a unique short (6-14 nucleotide) nucleic acid sequence within an adapter that can act as a 'key' to distinguish or separate a plurality of amplified target sequences in a sample. For the purposes of this disclosure, a DNA barcode or DNA tagging or index sequence can be incorporated into the nucleotide sequence of an adapter.

As used herein, the term "end" and its variants, when used in reference to a nucleic acid molecule, for example a target sequence or amplified target sequence, can include the terminal 30 nucleotides, the terminal 20 and even more typically the terminal 15 nucleotides of the nucleic acid molecule. A linear nucleic acid molecule comprised of linked series of contiguous nucleotides typically includes at least two ends. In some embodiments, one end of the nucleic acid molecule can include a 3' hydroxyl group or its equivalent, and can be referred to as the "3' end" and its derivatives. Optionally, the 3' end includes a 3' hydroxyl group that is not linked to a 5' phosphate group of a mononucleotide pentose ring. Typically, the 3' end includes one or more 5' linked nucleotides located adjacent to the nucleotide including the unlinked 3' hydroxyl group, typically the 30 nucleotides located adjacent to the 3' hydroxyl, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the unlinked 3' hydroxyl. For example, the 3' end can include less than 50% of the nucleotide length of the oligonucleotide. In some embodiments, the 3' end does not include any unlinked 3' hydroxyl group but can include any moiety capable of serving as a site for attachment of nucleotides via primer extension and/or nucleotide polymerization. In some embodiments, the term "3' end" for example when referring to a target-specific primer, can include the terminal 10 nucleotides, the terminal 5 nucleotides, the terminal 4, 3, 2 or fewer nucleotides at the 3'end. In some embodiments, the term "3' end" when referring to a target-specific primer can include nucleotides located at nucleotide positions 10 or fewer from the 3' terminus.

As used herein, "5' end", and its derivatives, generally refers to an end of a nucleic acid molecule, for example a target sequence or amplified target sequence, which includes a free 5' phosphate group or its equivalent. In some embodiments, the 5' end includes a 5' phosphate group that is not linked to a 3' hydroxyl of a neighboring mononucleotide pentose ring. Typically, the 5' end includes to one or more linked nucleotides located adjacent to the 5' phosphate, typically the 30 nucleotides located adjacent to the nucleotide including the 5' phosphate group, typically the terminal 20 and even more typically the terminal 15 nucleotides. Generally, the one or more linked nucleotides can be represented as a percentage of the nucleotides present in the oligonucleotide or can be provided as a number of linked nucleotides adjacent to the 5' phosphate. For example, the 5' end can be less than 50% of the nucleotide length of an oligonucleotide. In another exemplary embodiment, the 5' end can include about 15 nucleotides adjacent to the nucleotide including the terminal 5' phosphate. In some embodiments, the 5' end does not include any unlinked 5' phosphate group but can include any moiety capable of serving as a site of attachment to a 3' hydroxyl group, or to the 3'end of another nucleic acid molecule. In some embodiments, the term "5' end" for example when referring to a target-specific primer, can include the terminal 10 nucleotides, the terminal 5 nucleotides, the terminal 4, 3, 2 or fewer nucleotides at the 5'end. In some embodiments, the term "5' end" when referring to a target-specific primer can include nucleotides located at positions 10 or fewer from the 5' terminus. In some embodiments, the 5' end of a target-specific primer can include only non-cleavable nucleotides, for example nucleotides that do not contain one or more cleavable groups as disclosed herein, or a cleavable nucleotide as would be readily determined by one of ordinary skill in the art.

The term "extension" and its variants, as used herein, when used in reference to a given primer, comprises any in vivo or in vitro enzymatic activity characteristic of a given polymerase that relates to polymerization of one or more nucleotides onto an end of an existing nucleic acid molecule. Typically but not necessarily such primer extension occurs in a template-dependent fashion; during template-dependent extension, the order and selection of bases is driven by established base pairing rules, which can include Watson-Crick type base pairing rules or alternatively (and especially in the case of extension reactions involving nucleotide analogs) by some other type of base pairing paradigm. In one non-limiting example, extension occurs via polymerization of nucleotides on the 3'OH end of the nucleic acid molecule by the polymerase.

As used herein, the term "hybridization" is consistent with its use in the art, and generally refers to the process whereby two nucleic acid molecules undergo base pairing interactions. Two nucleic acid molecule molecules are said to be hybridized when any portion of one nucleic acid molecule is base paired with any portion of the other nucleic acid molecule; it is not necessarily required that the two nucleic acid molecules be hybridized across their entire respective lengths and in some embodiments, at least one of the nucleic acid molecules can include portions that are not hybridized to the other nucleic acid molecule. In some embodiments, conditions that are suitable for nucleic acid hybridization and/or for washing conditions include parameters such as salts, buffers, pH, temperature, GC% content of the polynucleotide and primers, and/or time. For example, conditions suitable for hybridizing or washing nucleic acids (e.g., polynucleotides and primers) can include hybridization solutions having sodium salts, such as NaCl, sodium citrate and/or sodium phosphate. In some embodiments, hybridization or wash solutions can include formamide (e.g., about 10-75%) and/or sodium dodecyl sulfate (SDS) (e.g., about 0.01-0.7%). In some embodiments, a hybridization solution can be a stringent hybridization solution which can include any combination of formamide (e.g., about 50%), 5 x SSC (e.g., about 0.75 M NaCl and about 0.075 M sodium citrate), sodium phosphate (e.g., about 50 mM at about pH 6.8), sodium pyrophosphate (e.g., about 0.1%), 5X Denhardt's solution, SDS (e.g., about 0.1%), and/or dextran sulfate (e.g., about 10%). In some embodiments, the hybridization or washing solution can include BSA (bovine serum albumin). In some embodiments, hybridization or washing can be conducted at a temperature range of about 15-25°C, or about 25-35°C, or about 35-45°C, or about 45-55°C, or about 55-65°C, or about 65-75°C, or about 75-85°C, or about 85-95°C, or about 95-99°C, or higher. In some embodiments, hybridization or washing can be conducted for a time range of about 1-10 minutes, or about 10-20 minutes, or about 20-30 minutes, or about 30-40 minutes, or about 40-50 minutes, or about 50-60 minutes, or longer. In some embodiments, hybridization or wash conditions can be conducted at a pH range of about 5-10, or about pH 6-9, or about pH 6.5-8, or about pH 6.5-7. Methods for nucleic acid hybridization and washing are well known in the art. For example, thermal melting temperature (Tm) for nucleic acids can be a temperature at which half of the nucleic acid strands are double-stranded and half are single-stranded under a defined condition. In some embodiments, a defined condition can include ionic strength and pH in an aqueous reaction condition. A defined condition can be modulated by altering the concentration of salts (e.g., sodium), temperature, pH, buffers, and/or formamide. Typically, the calculated thermal melting temperature can be at about 5-30° C below the Tm, or about 5-25° C below the Tm, or about 5-20° C below the Tm, or about 5-15° C below the Tm, or about 5-10° C below the Tm. Methods for calculating a Tm are well known and can be found in Sambrook (1989 in "Molecular Cloning: A Laboratory Manual", 2nd edition, volumes 1-3; Wetmur 1966, J. Mol. Biol., 31:349-370; Wetmur 1991 Critical Reviews in Biochemistry and Molecular Biology, 26:227-259). Other sources for calculating a Tm for hybridizing or denaturing nucleic acids include OligoAnalyze (from Integrated DNA Technologies) and Primer3 (distributed by the Whitehead Institute for Biomedical Research). The phrase "hybridizing under stringent conditions" and its variants refers generally to conditions under which hybridization of a target-specific primer to a target sequence occurs in the presence of high hybridization temperature and low ionic strength. In one exemplary embodiment, stringent hybridization conditions include an aqueous environment containing about 30 mM magnesium sulfate, about 300 mM Tris-sulfate at pH 8.9, and about 90 mM ammonium sulfate at about 60-68oC., or equivalents thereof. As used herein, the phrase "standard hybridization conditions" and its variants refers generally to conditions under which hybridization of a primer to an oligonucleotide (i.e., a target sequence), occurs in the presence of low hybridization temperature and high ionic strength. In one exemplary embodiment, standard hybridization conditions include an aqueous environment containing about 100 mM magnesium sulfate, about 500 mM Tris-sulfate at pH 8.9, and about 200 mM ammonium sulfate at about 50-55oC., or equivalents thereof.

The terms "identity" and "identical" and their variants, as used herein, when used in reference to two or more nucleic acid sequences, refer to similarity in sequence of the two or more sequences (e.g., nucleotide or polypeptide sequences). In the context of two or more homologous sequences, the percent identity or homology of the sequences or subsequences thereof indicates the percentage of all monomeric units (e.g., nucleotides or amino acids) that are the same (i.e., about 70% identity, preferably 75%, 80%, 85%, 90%, 95%, 98% or 99% identity). The percent identity can be over a specified region, when compared and aligned for maximum correspondence over a comparison window, or designated region as measured using a BLAST or BLAST 2.0 sequence comparison algorithms with default parameters described below, or by manual alignment and visual inspection. Sequences are said to be "substantially identical" when there is at least 85% identity at the amino acid level or at the nucleotide level. Preferably, the identity exists over a region that is at least about 25, 50, or 100 residues in length, or across the entire length of at least one compared sequence. A typical algorithm for determining percent sequence identity and sequence similarity are the BLAST and BLAST 2.0 algorithms, which are described in Altschul et al, Nuc. Acids Res. 25:3389-3402 (1977). Other methods include the algorithms of Smith & Waterman, Adv. Appl. Math. 2:482 (1981), and Needleman & Wunsch, J. Mol. Biol. 48:443 (1970), etc. Another indication that two nucleic acid sequences are substantially identical is that the two molecules or their complements hybridize to each other under stringent hybridization conditions.

As used herein, the terms "ligating", "ligation" and their derivatives refer generally to the act or process for covalently linking two or more molecules together, for example, covalently linking two or more nucleic acid molecules to each other. In some embodiments, ligation includes joining nicks between adjacent nucleotides of nucleic acids. In some embodiments, ligation includes forming a covalent bond between an end of a first and an end of a second nucleic acid molecule. In some embodiments, for example embodiments wherein the nucleic acid molecules to be ligated include conventional nucleotide residues, the ligation can include forming a covalent bond between a 5' phosphate group of one nucleic acid and a 3' hydroxyl group of a second nucleic acid thereby forming a ligated nucleic acid molecule. In some embodiments, any means for joining nicks or bonding a 5'phosphate to a 3' hydroxyl between adjacent nucleotides can be employed. In an exemplary embodiment, an enzyme such as a ligase can be used. Generally for the purposes of this disclosure, an amplified target sequence can be ligated to an adapter to generate an adapter-ligated amplified target sequence.

As used herein, "ligase" and its derivatives, refers generally to any agent capable of catalyzing the ligation of two substrate molecules. In some embodiments, the ligase includes an enzyme capable of catalyzing the joining of nicks between adjacent nucleotides of a nucleic acid. In some embodiments, the ligase includes an enzyme capable of catalyzing the formation of a covalent bond between a 5' phosphate of one nucleic acid molecule to a 3' hydroxyl of another nucleic acid molecule thereby forming a ligated nucleic acid molecule. Suitable ligases may include, but not limited to, T4 DNA ligase, T4 RNA ligase, and E. coli DNA ligase.

As used herein, "ligation conditions" and its derivatives, generally refers to conditions suitable for ligating two molecules to each other. In some embodiments, the ligation conditions are suitable for sealing nicks or gaps between nucleic acids. As defined herein, a "nick" or "gap" refers to a nucleic acid molecule that lacks a directly bound 5' phosphate of a mononucleotide pentose ring to a 3' hydroxyl of a neighboring mononucleotide pentose ring within internal nucleotides of a nucleic acid sequence. As used herein, the term nick or gap is consistent with the use of the term in the art. Typically, a nick or gap can be ligated in the presence of an enzyme, such as ligase at an appropriate temperature and pH. In some embodiments, T4 DNA ligase can join a nick between nucleic acids at a temperature of about 70-72oC.

As used herein, the term "nucleotide" and its variants comprises any compound, including without limitation any naturally occurring nucleotide or analog thereof, which can bind selectively to, or can be polymerized by, a polymerase. Typically, but not necessarily, selective binding of the nucleotide to the polymerase is followed by polymerization of the nucleotide into a nucleic acid strand by the polymerase; occasionally however the nucleotide may dissociate from the polymerase without becoming incorporated into the nucleic acid strand, an event referred to herein as a "non-productive" event. Such nucleotides include not only naturally occurring nucleotides but also any analogs, regardless of their structure, that can bind selectively to, or can be polymerized by, a polymerase. While naturally occurring nucleotides typically comprise base, sugar and phosphate moieties, the nucleotides of the present disclosure can include compounds lacking any one, some or all of such moieties. In some embodiments, the nucleotide can optionally include a chain of phosphorus atoms comprising three, four, five, six, seven, eight, nine, ten or more phosphorus atoms. In some embodiments, the phosphorus chain can be attached to any carbon of a sugar ring, such as the 5' carbon. The phosphorus chain can be linked to the sugar with an intervening O or S. In one embodiment, one or more phosphorus atoms in the chain can be part of a phosphate group having P and O. In another embodiment, the phosphorus atoms in the chain can be linked together with intervening O, NH, S, methylene, substituted methylene, ethylene, substituted ethylene, CNH2, C(O), C(CH2), CH2CH2, or C(OH)CH2R (where R can be a 4-pyridine or 1-imidazole). In one embodiment, the phosphorus atoms in the chain can have side groups having O, BH3, or S. In the phosphorus chain, a phosphorus atom with a side group other than O can be a substituted phosphate group. In the phosphorus chain, phosphorus atoms with an intervening atom other than O can be a substituted phosphate group. Some examples of nucleotide analogs are described in Xu, U.S. Patent No. 7,405,281. In some embodiments, the nucleotide comprises a label and referred to herein as a "labeled nucleotide"; the label of the labeled nucleotide is referred to herein as a "nucleotide label". In some embodiments, the label can be in the form of a fluorescent dye attached to the terminal phosphate group, i.e., the phosphate group most distal from the sugar. Some examples of nucleotides that can be used in the disclosed methods and compositions include, but are not limited to, ribonucleotides, deoxyribonucleotides, modified ribonucleotides, modified deoxyribonucleotides, ribonucleotide polyphosphates, deoxyribonucleotide polyphosphates, modified ribonucleotide polyphosphates, modified deoxyribonucleotide polyphosphates, peptide nucleotides, modified peptide nucleotides, metallonucleosides, phosphonate nucleosides, and modified phosphate-sugar backbone nucleotides, analogs, derivatives, or variants of the foregoing compounds, and the like. In some embodiments, the nucleotide can comprise non-oxygen moieties such as, for example, thio- or borano- moieties, in place of the oxygen moiety bridging the alpha phosphate and the sugar of the nucleotide, or the alpha and beta phosphates of the nucleotide, or the beta and gamma phosphates of the nucleotide, or between any other two phosphates of the nucleotide, or any combination thereof. "Nucleotide 5'-triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group can include sulfur substitutions for the various oxygens, e.g. .alpha.-thio-nucleotide 5'-triphosphates. For a review of nucleic acid chemistry, see: Shabarova, Z. and Bogdanov, A. Advanced Organic Chemistry of Nucleic Acids, VCH, New York, 1994.

As used herein, the term "nucleic acid" refers to natural nucleic acids, artificial nucleic acids, analogs thereof, or combinations thereof, including polynucleotides and oligonucleotides. As used herein, the terms "polynucleotide" and "oligonucleotide" are used interchangeably and mean single-stranded and double-stranded polymers of nucleotides including, but not limited to, 2'-deoxyribonucleotides (nucleic acid) and ribonucleotides (RNA) linked by internucleotide phosphodiester bond linkages, e.g. 3'-5' and 2'-5', inverted linkages, e.g. 3'-3' and 5'-5', branched structures, or analog nucleic acids. Polynucleotides have associated counter ions, such as H+, NH4+, trialkylammonium, Mg2+, Na+ and the like. An oligonucleotide can be composed entirely of deoxyribonucleotides, entirely of ribonucleotides, or chimeric mixtures thereof. Oligonucleotides can be comprised of nucleobase and sugar analogs. Polynucleotides typically range in size from a few monomeric units, e.g. 5-40, when they are more commonly frequently referred to in the art as oligonucleotides, to several thousands of monomeric nucleotide units, when they are more commonly referred to in the art as polynucleotides; for purposes of this disclosure, however, both oligonucleotides and polynucleotides may be of any suitable length. Unless denoted otherwise, whenever a oligonucleotide sequence is represented, it will be understood that the nucleotides are in 5' to 3' order from left to right and that "A" denotes deoxyadenosine, "C" denotes deoxycytidine, "G" denotes deoxyguanosine, "T" denotes thymidine, and "U' denotes deoxyuridine. Oligonucleotides are said to have "5' ends" and "3' ends" because mononucleotides are typically reacted to form oligonucleotides via attachment of the 5' phosphate or equivalent group of one nucleotide to the 3' hydroxyl or equivalent group of its neighboring nucleotide, optionally via a phosphodiester or other suitable linkage.

As used herein, "polymerase" and its derivatives, generally refers to any enzyme that can catalyze the polymerization of nucleotides (including analogs thereof) into a nucleic acid strand. Typically but not necessarily, such nucleotide polymerization can occur in a template-dependent fashion. Such polymerases can include without limitation naturally occurring polymerases and any subunits and truncations thereof, mutant polymerases, variant polymerases, recombinant, fusion or otherwise engineered polymerases, chemically modified polymerases, synthetic molecules or assemblies, and any analogs, derivatives or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases. Typically, the polymerase comprises one or more active sites at which nucleotide binding and/or catalysis of nucleotide polymerization can occur. Some exemplary polymerases include without limitation DNA polymerases and RNA polymerases. The term "polymerase" and its variants, as used herein, also refers to fusion proteins comprising at least two portions linked to each other, where the first portion comprises a peptide that can catalyze the polymerization of nucleotides into a nucleic acid strand and is linked to a second portion that comprises a second polypeptide. In some embodiments, the second polypeptide can include a reporter enzyme or a processivity-enhancing domain. Optionally, the polymerase can possess 5' exonuclease activity or terminal transferase activity. In some embodiments, the polymerase can be optionally reactivated, for example through the use of heat, chemicals or re-addition of new amounts of polymerase into a reaction mixture. In some embodiments, the polymerase can include a hot-start polymerase or an aptamer based polymerase that optionally can be reactivated.

As used herein, the term "polymerase chain reaction" ("PCR") refers to the method of K. B. Mullis U.S. Pat. Nos. 4,683,195 and 4,683,202, which describe a method for increasing the concentration of a segment of a polynucleotide of interest in a mixture of genomic DNA without cloning or purification. This process for amplifying the polynucleotide of interest consists of introducing a large excess of two oligonucleotide primers to the DNA mixture containing the desired polynucleotide of interest, followed by a precise sequence of thermal cycling in the presence of a DNA polymerase. The two primers are complementary to their respective strands of the double stranded polynucleotide of interest. To effect amplification, the mixture is denatured and the primers then annealed to their complementary sequences within the polynucleotide of interest molecule. Following annealing, the primers are extended with a polymerase to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired polynucleotide of interest. The length of the amplified segment of the desired polynucleotide of interest (amplicon) is determined by the relative positions of the primers with respect to each other, and therefore, this length is a controllable parameter. By virtue of repeating the process, the method is referred to as the "polymerase chain reaction" (hereinafter "PCR"). Because the desired amplified segments of the polynucleotide of interest become the predominant nucleic acid sequences (in terms of concentration) in the mixture, they are said to be "PCR amplified". As defined herein, target nucleic acid molecules within a sample including a plurality of target nucleic acid molecules are amplified via PCR. In a modification to the method discussed above, the target nucleic acid molecules can be PCR amplified using a plurality of different primer pairs, in some cases, one or more primer pairs per target nucleic acid molecule of interest, thereby forming a multiplex PCR reaction. Using multiplex PCR, it is possible to simultaneously amplify multiple nucleic acid molecules of interest from a sample to form amplified target sequences. It is also possible to detect the amplified target sequences by several different methodologies (e.g., quantitation with a bioanalyzer or qPCR, hybridization with a labeled probe; incorporation of biotinylated primers followed by avidin-enzyme conjugate detection; incorporation of 32P-labeled deoxynucleotide triphosphates, such as dCTP or dATP, into the amplified target sequence). Any oligonucleotide sequence can be amplified with the appropriate set of primers, thereby allowing for the amplification of target nucleic acid molecules from genomic DNA, cDNA, formalin-fixed paraffin-embedded DNA, fine-needle biopsies and various other sources. In particular, the amplified target sequences created by the multiplex PCR process as disclosed herein, are themselves efficient substrates for subsequent PCR amplification or various downstream assays or manipulations.

As used herein, "polymerizing conditions" and its derivatives, refers generally to conditions suitable for nucleotide polymerization. In typical embodiments, such nucleotide polymerization is catalyzed by a polymerase. In some embodiments, polymerizing conditions include conditions for primer extension, optionally in a template-dependent manner, resulting in the generation of a synthesized nucleic acid sequence. In some embodiments, the polymerizing conditions include polymerase chain reaction (PCR). Typically, the polymerizing conditions include use of a reaction mixture that is sufficient to synthesize nucleic acids and includes a polymerase and nucleotides. The polymerizing conditions can include conditions for annealing of a target-specific primer to a target sequence and extension of the primer in a template dependent manner in the presence of a polymerase. In some embodiments, polymerizing conditions can be practiced using thermocycling. Additionally, polymerizing conditions can include a plurality of cycles where the steps of annealing, extending, and separating the two nucleic strands are repeated. Typically, the polymerizing conditions include a cation such as MgCl2. Generally, polymerization of one or more nucleotides to form a nucleic acid strand includes that the nucleotides be linked to each other via phosphodiester bonds, however, alternative linkages may be possible in the context of particular nucleotide analogs.

The term "portion" and its variants, as used herein, when used in reference to a given nucleic acid molecule, for example a primer or a template nucleic acid molecule, comprises any number of contiguous nucleotides within the length of the nucleic acid molecule, including the partial or entire length of the nucleic acid molecule.

As used herein, "protecting group" and its derivatives, refers generally to any moiety that can be incorporated into an adapter or target-specific primer that imparts chemical selectivity or protects the target-specific primer or adapter from digestion or chemical degradation. Typically, but not necessarily, a protecting group can include modification of an existing functional group in the target-specific primer r adapter to achieve chemical selectivity. Suitable types of protecting groups include alcohol, amine, phosphate, carbonyl, or carboxylic acid protecting groups. In an exemplary embodiment, the protecting group can include a spacer compound having a chain of carbon atoms.

As defined herein, "sample" and its derivatives, is used in its broadest sense and includes any specimen, culture and the like that is suspected of including a target. In some embodiments, the sample comprises DNA, RNA, PNA, LNA, chimeric, hybrid, or multiplex-forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen.

As used herein, "synthesizing" and its derivatives, refers generally to a reaction involving nucleotide polymerization by a polymerase, optionally in a template-dependent fashion. Polymerases synthesize an oligonucleotide via transfer of a nucleoside monophosphate from a nucleoside triphosphate (NTP), deoxynucleoside triphosphate (dNTP) or dideoxynucleoside triphosphate (ddNTP) to the 3' hydroxyl of an extending oligonucleotide chain. For the purposes of this disclosure, synthesizing includes to the serial extension of a hybridized adapter or a target-specific primer via transfer of a nucleoside monophosphate from a deoxynucleoside triphosphate.

As used herein, "target sequence" or "target sequence of interest" and its derivatives, refers generally to any single or double-stranded nucleic acid sequence that can be amplified or synthesized according to the disclosure, including any nucleic acid sequence suspected or expected to be present in a sample. In some embodiments, the target sequence is present in double-stranded form and includes at least a portion of the particular nucleotide sequence to be amplified or synthesized, or its complement, prior to the addition of target-specific primers or appended adapters. Target sequences can include the nucleic acids to which primers useful in the amplification or synthesis reaction can hybridize prior to extension by a polymerase. In some embodiments, the term refers to a nucleic acid sequence whose sequence identity, ordering or location of nucleotides is determined by one or more of the methods of the disclosure.

Library prepared according to the provided methods can be used in many downstream analysis or assays with, or without, further purification or manipulation. For example, the library products when obtained in sufficient yield can be used for single nucleotide polymorphism (SNP) analysis, genotyping, copy number variation analysis, epigenetic analysis, gene expression analysis, hybridization arrays, analysis of gene mutations including but not limited to detection, prognosis and/or diagnosis of disease states, detection and analysis of rare or low frequency allele mutations, nucleic acid sequencing including but not limited to de novo sequencing or targeted resequencing, and the like.

In some embodiments, the library produced by the teachings of the present disclosure are sufficient in yield to be used in a variety of downstream application. For example, the Ion Xpress^{™} Template Kit using an Ion Torrent^{™} PGM system (e.g., PCR-mediated addition of the nucleic acid fragment library onto Ion Sphere^{™} Particles)(Life Technologies, Part No. 4467389), instructions to prepare a template library from the amplicon library can be found in the Ion Xpress Template Kit User Guide (Life Technologies, Part No. 4465884).

In some embodiments, the disclosure generally relates to methods for preparing a target-specific amplicon library, for use in a variety of downstream processes or assays such as nucleic acid sequencing or clonal amplification. In some embodiments, prepared library is optionally manipulated or amplified through bridge amplification or clonal amplification such as emPCR to generate a plurality of clonal templates that are suitable for a variety of downstream processes including nucleic acid sequencing. In some embodiments, at least one of the amplified targets sequences to be clonally amplified can be attached to a support or particle. The support can be comprised of any suitable material and have any suitable shape, including, for example, planar, spheroid or particulate. In some embodiments, the support is a scaffolded polymer particle as described in U.S. Published App. No. 20100304982. It is also envisaged that one of ordinary skill in art upon further refinement or optimization of the conditions provided herein can proceed directly to nucleic acid sequencing without performing a clonal amplification step.

Following library preparation, the adapter-target-adapters or library of nucleic acids can be sequenced. Sequencing can be carried out by a variety of known methods, including, but not limited to sequencing by synthesis, sequencing by ligation, and/or sequencing by hybridization.

Sequencing by synthesis, for example, is a technique wherein nucleotides are added successively to a free 3' hydroxyl group, typically provided by annealing of an oligonucleotide primer (e.g., a sequencing primer), resulting in synthesis of a nucleic acid chain in the 5' to 3' direction. These and other sequencing reactions may be conducted on the herein described surfaces bearing nucleic acid clusters. The reactions comprise one or a plurality of sequencing steps, each step comprising determining the nucleotide incorporated into a nucleic acid chain and identifying the position of the incorporated nucleotide on the surface. The nucleotides incorporated into the nucleic acid chain may be described as sequencing nucleotides and may comprise one or more detectable labels. Suitable detectable labels, include, but are not limited to, haptens, radionucleotides, enzymes, fluorescent labels, chemiluminescent labels, and/or chromogenic agents. One method for detecting fluorescently labeled nucleotides comprises using laser light of a wavelength specific for the labeled nucleotides, or the use of other suitable sources of illumination. The fluorescence from the label on the nucleotide may be detected by a CCD camera or other suitable detection means. Suitable instrumentation for recording images of clustered arrays is described in WO 07/123744.

Optionally, cycle sequencing is accomplished by stepwise addition of reversible terminator nucleotides containing, for example, a cleavable or photobleachable dye label as described, for example, in U.S. Patent No. 7,427,673; U.S. Patent No. 7,414, 116; WO 04/018497; WO 91/06678; WO 07/123744; and U.S. Patent No. 7,057,026. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Alternatively, pyrosequencing techniques may be employed. Pyrosequencing detects the release of inorganic pyrophosphate (PPi) as particular nucleotides are incorporated into the nascent strand (Ronaghi et al, (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 1 1(1), 3-11 ; Ronaghi, M., Uhlen, M. and Nyren, P. (1998) "A sequencing method based on real-time pyrophosphate." Science 281(5375), 363; U.S. Patent No. 6,210,891 ; U.S. Patent No. 6,258,568; and U.S. Patent No. 6,274,320). In pyrosequencing, released PPi can be detected by being immediately converted to adenosine triphosphate (ATP) by ATP sulfurylase, and the level of ATP generated is detected via luciferase-produced photons.

Additionally, ion-based sequencing systems sequence nucleic acid templates by detecting ions produced as a byproduct of nucleotide incorporation. Typically, hydrogen ions are released as byproducts of nucleotide incorporations occurring during template-dependent nucleic acid synthesis by a polymerase. The Ion Torrent PGM^{™} sequencer and Ion Proton ^{™} Sequencer detect the nucleotide incorporations by detecting the hydrogen ion byproducts of the nucleotide incorporations. The Ion Torrent PGM^{™} sequencer and Ion Torrent Proton^{™} sequencer include a plurality of nucleic acid templates to be sequenced, each template disposed within a respective sequencing reaction well in an array. The wells of the array are each coupled to at least one ion sensor that can detect the release of H+ ions or changes in solution pH produced as a byproduct of nucleotide incorporation. The ion sensor comprises a field effect transistor (FET) coupled to an ion-sensitive detection layer that can sense the presence of H+ ions or changes in solution pH. The ion sensor provides output signals indicative of nucleotide incorporation which can be represented as voltage changes whose magnitude correlates with the H+ ion concentration in a respective well or reaction chamber. Different nucleotide types are flowed serially into the reaction chamber, and are incorporated by the polymerase into an extending primer (or polymerization site) in an order determined by the sequence of the template. Each nucleotide incorporation is accompanied by the release of H+ ions in the reaction well, along with a concomitant change in the localized pH. The release of H+ ions is registered by the FET of the sensor, which produces signals indicating the occurrence of the nucleotide incorporation. Nucleotides that are not incorporated during a particular nucleotide flow will not produce signals. The amplitude of the signals from the FET may also be correlated with the number of nucleotides of a particular type incorporated into the extending nucleic acid molecule thereby permitting homopolymer regions to be resolved. Thus, during a run of the sequencer multiple nucleotide flows into the reaction chamber along with incorporation monitoring across a multiplicity of wells or reaction chambers permit the instrument to resolve the sequence of many nucleic acid templates simultaneously. Further details regarding the compositions, design and operation of the Ion Torrent PGM^{™} sequencer can be found, for example, in U.S. Patent Publication No. 2009/0026082; U.S. Patent Publication No. 2010/0137143; and U.S. Patent Publication No. 2010/0282617. Instructions for loading the subsequent template library onto the Ion Torrent^{™} Chip for nucleic acid sequencing are described in the Ion Sequencing User Guide (Part No. 4467391). In some embodiments, the amplicon library produced by the teachings of the present disclosure can be used in paired end sequencing (e.g., paired-end sequencing on the Ion Torrent^{™} PGM system (Life Technologies, Part No. MAN0006191).

Additional exemplary sequencing-by-synthesis methods that can be used with the methods described herein include those described in U.S. Patent Publication Nos. 2007/0166705; 2006/0188901 ; 2006/0240439; 2006/0281 109; 2005/0100900; U.S. Patent No. 7057026; WO 05/065814; WO 06/064199; WO 07/010251.

Alternatively, sequencing by ligation techniques are used. Such techniques use DNA ligase to incorporate oligonucleotides and identify the incorporation of such oligonucleotides and are described in U.S. Patent No 6,969,488; U.S. Patent No. 6, 172,218; and U.S. Patent No. 6,306,597. Other suitable alternative techniques include, for example, fluorescent in situ sequencing (FISSEQ), and Massively Parallel Signature Sequencing (MPSS).

**TABLE 1: SEQUENCE LISTING**

| SEQ ID. | SEQUENCE |
|---|---|
| 1 | |
| 2) | |
| 3 | 5'CTGTCTCTTATACACATCTCCGAGCCCACGAGAC |
| 4 | 5'CTGTCTCTTATACACATCTCCGAGCCCACGAGAC-Amino |
| 5 | 5'CTGTCTCTTATACACATCTGACGCTGCCGAC*G*A |
| 6) | |
| 7 | |
| 8 | 5'CTGTCTCTTATACACATCTGACGCTGCCGACGA |
| 9 | 5'CTGTCTCTTATACACATCTGACGCTGCCGACGA-Amino |
| 10 | 5'CTGTCTCTTATACACATCTGACGCTGCCGAC*G*A |

SEQ ID NO: 1 - N701 (forward adapter sequence); SEQ ID NO: 2 - N701_rc (full length reverse complement); SEQ ID NO: 3 - N701_rc_34 (unprotected 3' end); SEQ ID NO:4 - N701_rc_34Am (Amino modified 3' end); SEQ ID NO:5 - N701_rc_34PT (Phosphorothioate modified 3' end); SEQ ID NO:6 - S502 (forward adapter sequence); SEQ ID NO:7 - S502_rc (full length reverse complement); SEQ ID NO:8 - S502_rc_33 (unprotected 3' end); SEQ ID NO:9 - S502_rc_33Am (Amino modified 3' end); SEQ ID NO:10 - S502_rc_33PT (Phosphorothioate modified 3' end)

### EXEMPLIFICATION

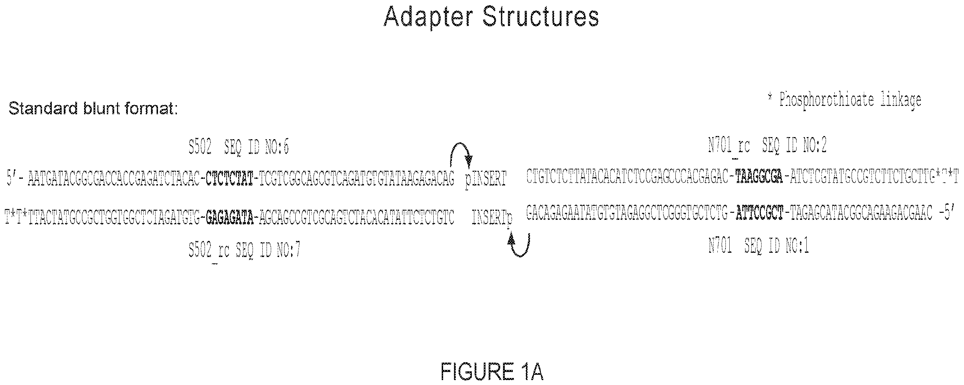

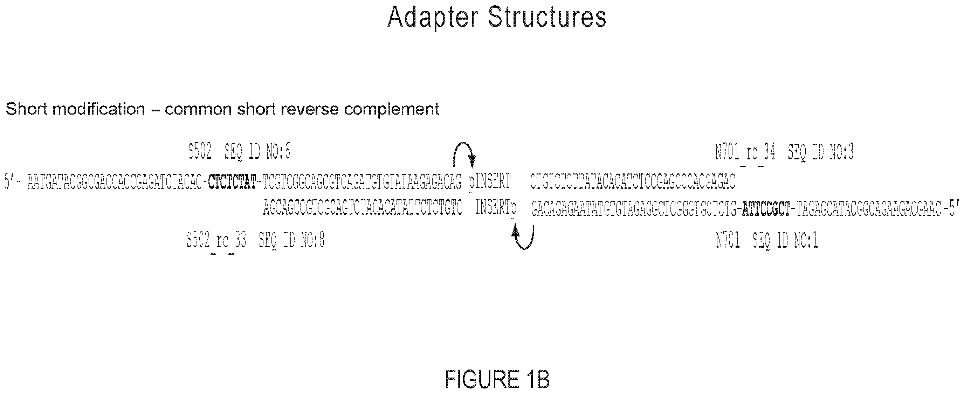

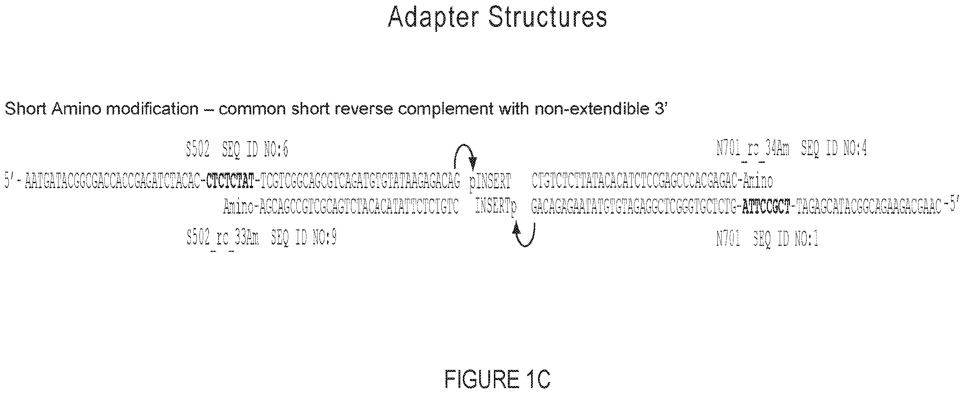

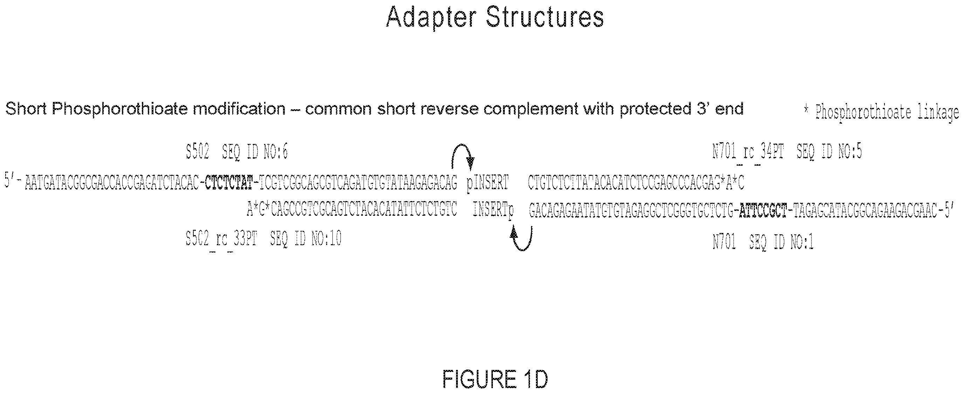

Universal reverse complement sequences were designed to the 3' region of the barcode of the Nextera XT v2 adapter structures. For example, one strand complementary to N701 and one to S502 (Illumina, Inc.). Three configuration formats were prepared for each: an unprotected 3' end, a non-extendable 3' amino modifier, and phosphorothioate protection of the 2 3' bases. See TABLE 1, FIG. 1. The shorter complementary structures prepared resulted in an approximately 10-fold reduction in adapter dimer formation, equivalent to higher library yield, and equivalent to better sequencing performance.

Illumina Nextera XT v2 adapters use a dual index system which consists of 2 barcodes i7 (Index Read 1, exemplified herein N701) and i5 (Index Read 2, exemplified herein S502). See, e.g., TABLE 2. We designed full length reverse complement sequences with 3' phosphorothioate protected overhang, as well as short reverse complement sequences, e.g., we used an unmodified full length sequence paired with 34-base (N701) or 33-base (S502) complement. While N701 and S502 Indexes have been used herein, any of the i7 and/or i5 indexes can be used in conjunction with compositions and methods provided herein. The 34/33 base sequences are common to all N7xx and S5xx sequences in the Nextera family respectively, allowing us to synthesize a single reverse complement for each index type. Unmodified, 3' amino modified and 3' phosphorothioate protected versions of shortened complement sequences were prepared. In the case of unmodified and 3' phosphorothioate versions, adapters could potentially be blunted by residual polymerase activity, leading to production of increased dimer formation, while the 3' amino modified version is non-extendable. When used with the 34/33 reverse complements, full length forward adapter has a 32 or 37-base unprotected 5' overhang.

**TABLE 2: Nextera XT Index Kit v2 Sequences -**

| | | | | | |
|---|---|---|---|---|---|
| **A PCR Primers** | | | | | |
| Read 1: 5' TCGTCGGCAGCGTCAGATGTGTATAAGAGACAG (SEQ ID NO:11) | | | | | |
| Index 1 Read: 5' CAAGCAGAAGACGGCATACGAGAT[i7]GTCTCGTGGGCTCGG (SEQ ID NO: 12) | | | | | |
| Read 2L 5' GTCTCGTGGGCTCGGAGATGTGTATAAGAGACAG (SEQ ID NO:13) | | | | | |
| Index 2 Read: 5' AATGATACGGCGACCACCGAGATCTACAC[i5]TCGTCGGCAGCGTC (SEQ ID NO:14) | | | | | |
| **B Index 1 (i7) and Index 2 (i5)** | | | | | |

| Bases in Adapter | i7 Index | SEQ ID | Bases in Adapter | i5 Index | SEQ ID |
|---|---|---|---|---|---|
| TCGCCTTA | N701 | 15 | CTCTCTAT | S502 | 39 |
| CTAGTACG | N702 | 16 | TATCCTCT | S503 | 40 |
| TTCTGCCT | N703 | 17 | GTAAGGAG | S505 | 41 |
| GCTCAGGA | N704 | 18 | ACTGCATA | S506 | 42 |
| AGGAGTCC | N705 | 19 | AAGGAGTA | S507 | 43 |
| CATGCCTA | N706 | 20 | CTAAGCCT | S508 | 44 |
| GTAGAGAG | N707 | 21 | CGTCTAAT | S510 | 45 |
| CAGCCTCG | N710 | 22 | TCTCTCCG | S511 | 46 |
| TGCCTCTT | N711 | 23 | TCGACTAG | S513 | 47 |
| TCCTCTAC | N712 | 24 | TTCTAGCT | S515 | 48 |
| TCATGAGC | N714 | 25 | CCTAGAGT | S516 | 49 |
| CCTGAGAT | N715 | 26 | GCGTAAGA | S517 | 50 |
| TAGCGAGT | N716 | 27 | CTATTAAG | S518 | 51 |
| GTAGCTCC | N718 | 28 | AAGGCTAT | S520 | 52 |
| TACTACGC | N719 | 29 | GAGCCTTA | S521 | 53 |
| AGGCTCCG | N720 | 30 | TTATGCGA | S522 | 54 |
| GCAGCGTA | N721 | 31 | | | |
| CTGCGCAT | N722 | 32 | | | |
| GAGCGCTA | N723 | 33 | | | |
| CGCTCAGT | N724 | 34 | | | |
| GTCTTAGG | N726 | 35 | | | |
| ACTGATCG | N727 | 36 | | | |
| TAGCTGCA | N728 | 37 | | | |
| GACGTCGA | N729 | 38 | | | |

| | | | | | |
|---|---|---|---|---|---|
| Oligonucleotide sequences Ⓒ 2016 Illumina, Inc. All rights reserved. | | | | | |

To evaluate adapter configurations, barcodes were annealed using existing annealing protocols consisting of a 90C denaturation for 5min followed by a slow cooling (30sec/1C) to 25C. Annealed adapters were diluted to working concentration (10uM) and 1uL each index was used in ligation reactions with amplicon pools for library generation. Prepared adapters were ligated to amplicon pools prepared using the Ion AmpliSeqTM Exome RDY Kit (Thermo Fisher Scientific) according to manufacturer instructions. Ligation reactions were carried out as described in the AmpliSeqTM workflow protocol. Performance was evaluated by qPCR, Bioanalyzer, and sequencing on a MiSeq (Illumina Inc.), according to manufacturer instructions.

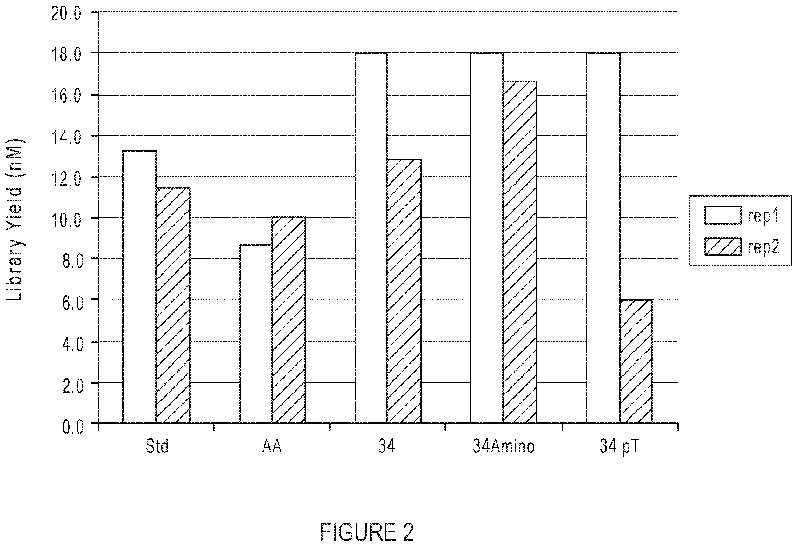

As mentioned, adapter configurations were evaluated for performance by qPCR, Bioanalyzer and sequencing. Library quantitation by qPCR showed a 30-40% yield improvement with short adapters compared to full length. See FIG. 2.

Adapter dimer formation was analyzed on a Bioanalyzer and saw a 80-90% reduction in the amount of dimer produced during ligation when a short reverse complement sequence is used. See TABLE 2. Quantification of the peak intensities demonstrated this reduction is nearly 10-fold for all short sequences. A modified full length adapter (AA) shows similar, high dimer formation compared to standard full length adapter (std). See TABLE 3.

**TABLE 3: DIMER FORMATION**

| Exome Pool | Adapter | Dimer (pM) | Library (pM) | Dimer % | Δ Dimer vs STD |
|---|---|---|---|---|---|
| 1 | std | 1853 | 9562 | 16.2% | 0% |
| 2 | AA | 1700 | 5817 | 22.6% | 39% |
| 3 | 34 | 283 | 13415 | 2.1% | -87% |
| 4 | 34 Amino | 297 | 15894 | 1.8% | -89% |
| 5 | 34 pT | 129.7 | 13949 | 0.9% | -94% |
| 6 | std | 1395 | 7210 | 16.2% | 0% |
| 7 | AA | 1734 | 7759 | 18.3% | 13% |
| 8 | 34 | 419.9 | 10757 | 3.8% | -77% |
| 9 | 34 Amino | 259.8 | 13642 | 1.9% | -88% |
| 10 | 34 pT | 98.3 | 5639 | 1.7% | -89% |

The short adapter structure results in a long 5' unprotected overhang that has potential for degradation. We developed assays to confirm that the overhang is not digested by reagents present in the ligation reactions. For example, to examine degradation, reaction enzyme activity was heat killed for 20min at 60C, adapter added, then digested for 60min at 37C. Degradation measured by gel electrophoresis.confirmed that under a variety of various conditions of buffers, enzymes, and temperatures, an unmodified 5' end is not digested.

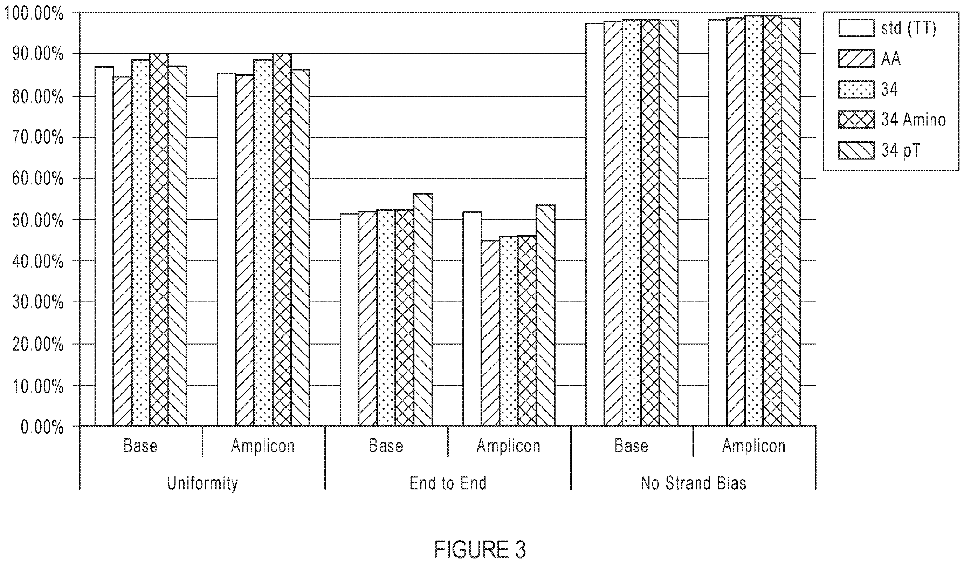

Prepared libraries were sequenced on a MiSeq and performance metrics were equivalent with shorter sequences. See FIG 3. Provided modified adapters have equivalent sequencing performance to standard adapters. We saw no significant differences in these sequencing metrics (uniformity, end to end sequencing, strand bias).

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method of reducing adapter dimer formation comprising contacting a sample comprising target nucleic acid sequences with 5 ' and 3 ' adapters under conditions to form 5'-adapter-target-3'-adapter sequences, wherein the amount of adapter dimer formation is reduced compared to the amount in the absence of the oligonucleotides;
wherein the 5' and 3' adapters each comprise short reverse complementary sequences over less than seventy percent (70%) of the adapter length at its 3' end.

2. A method of preparing a library of nucleic acid sequences comprising: contacting first and second adapter oligonucleotides with a sample comprising target nucleic acid sequences under conditions to form to form 5'-adapter-target-3'-adapter ligation products; wherein the adapter oligonucleotides comprise 5 ' and 3 ' adapters each having short reverse complementary sequences over less than seventy percent (70%) of the adapter length at its 3' end and form ligation products, wherein the ligation products form the library of nucleic acid sequences; optionally
further comprising amplifying the ligation products to product the library of nucleic acid sequences; and optionally.
further comprising sequencing the ligation products,
wherein adapter dimer formation is reduced compared to the amount of adapter dimer formation in the presence of adapter oligonucleotides having full length reverse complementary sequence.

3. The method of claim 1or 2, wherein less than 25, 20, 15, 10, 8, 6, 5, 4, 3, 2, or 1% of adapters form dimers.

4. The method of claim 1 or 2, wherein the 5' and/or 3' adapters each comprise short reverse complementary sequences over less than 65, 60, 55, 50, 45, or 40 percent of the length at its 3' end.

5. The method of claim 1 or 2, wherein the adapters comprise single stranded 5' overhang, optionally wherein the adapters comprise 5' overhang sequences over at least 30, 40, 45, 50, 55, or 60 percent of the length.

6. The method of claim 1 or 2, wherein the adapters comprise modified sequences.

7. The method of claim 6, wherein the adapter is a 3'-phosphorothioate protected adapter, or a 3'-amino modified adapter.

8. The method of claim 1 or 2, wherein the 5' and 3' adapters are linear, are blunt ended or comprise T overhangs.at their 3' end.

9. A composition comprising an adapter sequence having a 5' extended overhang sequence and a 3' blunt end or T overhang sequence, wherein the adapter comprises short reverse complementary sequences over less than 60, 55, 50, 45, or 40 percent of the length at its 3' end.

10. The composition of claim 9 comprising a 5' adapter sequence and a 3' adapter sequence capable of ligating to amplicon target sequences of interest.

11. The method of claim 1 or 2 or the composition of claim 9 wherein the reverse complement oligonucleotide adapter sequence is selected from the group consisting of SEQ ID NO:3, 4, or 5 or selected from the group consisting of SEQ ID NO:7, 8, or 9.

12. The method of claim 1 or 2 or the composition of claim 9, wherein the forward oligonucleotide adapter sequence comprises SEQ ID NO:1 and wherein the reverse complement oligonucleotide adapter sequence is selected from the group consisting of SEQ ID NO:3, 4, or 5

13. The method of claim 1 or 2 or the composition of claim 9, wherein the forward oligonucleotide adapter sequence comprises SEQ ID NO:6 and wherein the reverse complement oligonucleotide adapter sequence is selected from the group consisting of SEQ ID NO:7, 8, or 9.

14. The method of claim 2, wherein the oligonucleotide adapters are not complementary to the target nucleic acid sequences and wherein the target nucleic acid molecules comprise different sequences.

15. The method of claim 2, wherein the first and second adapters have universal sequences.

## Patentansprüche

1. Verfahren zum Reduzieren einer Adapterdimerausbildung, umfassend ein Inberührungbringen einer Probe, die Zielnukleinsäuresequenzen umfasst, mit 5'- und 3'-Adaptern unter Bedingungen, um 5'-Adapter-Ziel-3'-Adaptersequenzen auszubilden, wobei die Menge der Adapterdimerausbildung verglichen mit der Menge in Abwesenheit der Oligonukleotide reduziert ist;
wobei die 5'- und 3'-Adapter jeweils kurze revers komplementäre Sequenzen über weniger als siebzig Prozent (70 %) der Adapterlänge an ihrem 3'-Ende umfassen.

2. Verfahren zum Herstellen einer Bibliothek von Nukleinsäuresequenzen, umfassend:
Inberührungbringen erster und zweiter Adapteroligonukleotide mit einer Probe, die Zielnukleinsäuresequenzen umfasst, unter Bedingungen, um 5'-Adapter-Ziel-3'-Adapter Ligationsprodukte auszubilden;
wobei die Adapteroligonukleotide 5'- und 3'-Adapter umfassen, die jeweils kurze revers komplementäre Sequenzen über weniger als siebzig Prozent (70 %) der Adapterlänge an ihrem 3'-Ende aufweisen und Ligationsprodukte ausbilden, wobei die Ligationsprodukte die Bibliothek von Nukleinsäuresequenzen ausbilden; optional ferner umfassend ein Amplifizieren der Ligationsprodukte, um die Bibliothek von Nukleinsäuresequenzen zu erstellen; und optional.
ferner umfassend ein Sequenzieren der Ligationsprodukte,
wobei die Adapterdimerausbildung verglichen mit der Menge der Adapterdimerausbildung in der Gegenwart von Adapteroligonukleotiden, die eine revers komplementäre Sequenz voller Länge aufweisen, reduziert ist.

3. Verfahren nach Anspruch 1 oder 2, wobei weniger als 25, 20, 15, 10, 8, 6, 5, 4, 3, 2 oder 1 % der Adapter Dimere ausbilden.

4. Verfahren nach Anspruch 1 oder 2, wobei die 5'- und/oder 3'-Adapter jeweils kurze revers komplementäre Sequenzen über weniger als 65, 60, 55, 50, 45 oder 40 Prozent der Länge an ihrem 3'-Ende umfassen.

5. Verfahren nach Anspruch 1 oder 2, wobei die Adapter einzelsträngigen 5'-Überhang umfassen, optional wobei die Adapter 5'-Überhangsequenzen über wenigstens 30, 40, 45, 50, 55 oder 60 Prozent der Länge umfassen.

6. Verfahren nach Anspruch 1 oder 2, wobei die Adapter modifizierte Sequenzen umfassen.

7. Verfahren nach Anspruch 6, wobei der Adapter ein 3'-Phosphorothioat-geschützter Adapter oder ein 3'-Amino-modifizierter Adapter ist.

8. Verfahren nach Anspruch 1 oder 2, wobei die 5'- und 3'-Adapter linear sind, stumpfe Enden aufweisen oder T-Überhänge an ihrem 3'-Ende umfassen.

9. Zusammensetzung, umfassend eine Adaptersequenz, die eine 5'-verlängerte Überhangsequenz und ein 3'-stumpfes Ende oder eine T-Überhangsequenz aufweist, wobei der Adapter kurze revers komplementäre Sequenzen über weniger als 60, 55, 50, 45 oder 40 Prozent der Länge an seinem 3'-Ende umfasst.

10. Zusammensetzung nach Anspruch 9, umfassend eine 5'-Adaptersequenz und eine 3'-Adaptersequenz, die in der Lage sind, mit Amplikonzielsequenzen von Interesse zu ligieren.

11. Verfahren nach Anspruch 1 oder 2 oder die Zusammensetzung nach Anspruch 9, wobei die reverse Komplement-Oligonukleotidadaptersequenz aus der Gruppe ausgewählt ist, die aus SEQ ID NO: 3, 4 oder 5 besteht oder aus der Gruppe ausgewählt ist, die aus SEQ ID NO: 7, 8, oder 9 besteht.

12. Verfahren nach Anspruch 1 oder 2 oder die Zusammensetzung nach Anspruch 9, wobei die Vorwärts-Oligonukleotidadaptersequenz SEQ ID NO: 1 umfasst und wobei die reverse Komplement-Oligonukleotidadaptersequenz aus der Gruppe ausgewählt ist, die aus SEQ ID NO: 3, 4 oder 5 besteht.

13. Verfahren nach Anspruch 1 oder 2 oder Zusammensetzung nach Anspruch 9, wobei die Vorwärts-Oligonukleotidadaptersequenz SEQ ID NO: 6 umfasst und wobei die reverse Komplement-Oligonukleotidadaptersequenz aus der Gruppe ausgewählt ist, die aus SEQ ID NO: 7, 8 oder 9 besteht.

14. Verfahren nach Anspruch 2, wobei die Oligonukleotidadapter nicht komplementär zu den Zielnukleinsäuresequenzen sind und wobei die Zielnukleinsäuremoleküle unterschiedliche Sequenzen umfassen.

15. Verfahren nach Anspruch 2, wobei der erste und der zweite Adapter universelle Sequenzen aufweisen.

## Revendications

1. Procédé destiné à la réduction de la formation de dimères d'adaptateurs comprenant la mise en contact d'un échantillon comprenant des séquences d'acides nucléiques cibles avec des adaptateurs 5' et 3' dans des conditions pour former des séquences adaptateur 5'-cible-adaptateur 3', la quantité de formation de dimères d'adaptateurs étant réduite par rapport à la quantité en l'absence des oligonucléotides ;
les adaptateurs 5' et 3' comprenant chacun de courtes séquences complémentaires inverses sur moins de soixante-dix pour cent (70 %) de la longueur de l'adaptateur à son extrémité 3'.

2. Procédé destiné à la préparation d'une banque de séquences d'acides nucléiques comprenant : la mise en contact de premier et second oligonucléotides adaptateurs avec un échantillon comprenant des séquences d'acides nucléiques cibles dans des conditions pour former des produits de ligature adaptateur 5'-cible-adaptateur 3' ;
les oligonucléotides adaptateurs comprenant des adaptateurs 5' et 3' ayant chacun de courtes séquences complémentaires inverses sur moins de soixante-dix pour cent (70 %) de la longueur de l'adaptateur à son extrémité 3' et formant des produits de ligature, les produits de ligature formant la banque de séquences d'acides nucléiques ; éventuellement
comprenant en outre l'amplification des produits de ligature pour produire la banque de séquences d'acides nucléiques ; et éventuellement
comprenant en outre le séquençage des produits de ligature,
la formation de dimères d'adaptateurs étant réduite par rapport à la quantité de formation de dimères d'adaptateurs en présence d'oligonucléotides adaptateurs ayant une séquence complémentaire inverse sur toute la longueur.

3. Procédé selon la revendication 1 ou 2, dans lequel moins de 25, 20, 15, 10, 8, 6, 5, 4, 3, 2 ou 1 % d'adaptateurs forment des dimères.

4. Procédé selon la revendication 1 ou 2, dans lequel les adaptateurs 5' et/ou 3' comprennent chacun de courtes séquences complémentaires inverses sur moins de 65, 60, 55, 50, 45 ou 40 pour cent de la longueur à leur extrémité 3'.

5. Procédé selon la revendication 1 ou 2, dans lequel les adaptateurs comprennent un surplomb 5' simple brin, éventuellement dans lequel les adaptateurs comprennent des séquences de surplomb 5' sur au moins 30, 40, 45, 50, 55 ou 60 pour cent de la longueur.

6. Procédé selon la revendication 1 ou 2, dans lequel les adaptateurs comprennent des séquences modifiées.

7. Procédé selon la revendication 6, dans lequel l'adaptateur est un adaptateur protégé par 3'-phosphorothioate, ou un adaptateur modifié par 3'-amino.

8. Procédé selon la revendication 1 ou 2, dans lequel les adaptateurs 5' et 3' sont linéaires, ont des extrémités franches ou comprennent des surplombs T à leur extrémité 3'.

9. Composition comprenant une séquence d'adaptateur ayant une séquence de surplomb étendue 5' et une extrémité franche 3' ou une séquence de surplomb T, l'adaptateur comprenant de courtes séquences complémentaires inverses sur moins de 60, 55, 50, 45 ou 40 pour cent de la longueur à son extrémité 3'.

10. Composition selon la revendication 9, comprenant une séquence d'adaptateur 5' et une séquence d'adaptateur 3' capables de se ligaturer à des séquences cibles d'intérêt d'amplicon.

11. Procédé selon la revendication 1 ou 2 ou composition selon la revendication 9, dans lequel la séquence d'adaptateur d'oligonucléotide complémentaire inverse est choisie dans le groupe constitué par SEQ ID NO : 3, 4 ou 5 ou choisie dans le groupe constitué par SEQ ID NO : 7, 8, ou 9.

12. Procédé selon la revendication 1 ou 2 ou composition selon la revendication 9, dans lequel la séquence d'adaptateur d'oligonucléotide avant comprend SEQ ID NO : 1 et dans lequel la séquence d'adaptateur d'oligonucléotide complémentaire inverse est choisie dans le groupe constitué par SEQ ID NO : 3, 4, ou 5.

13. Procédé selon la revendication 1 ou 2 ou composition selon la revendication 9, dans lequel la séquence d'adaptateur d'oligonucléotide avant comprend SEQ ID NO : 6 et dans lequel la séquence d'adaptateur d'oligonucléotide complémentaire inverse est choisie dans le groupe constitué par SEQ ID NO : 7, 8 ou 9.

14. Procédé selon la revendication 2, dans lequel les adaptateurs d'oligonucléotides ne sont pas complémentaires des séquences d'acides nucléiques cibles et dans lequel les molécules d'acides nucléiques cibles comprennent des séquences différentes.

15. Procédé selon la revendication 2, dans lequel les premier et second adaptateurs ont des séquences universelles.


### SEQUENCE LISTING

<110> LIFE TECHNOLOGIES CORPORATION
   ANDERSEN, Mark
   ALLEN, Michael
<120> POLYNUCLEOTIDE ADAPTERS AND METHODS OF USE THEREOF
<130> LT01237US
<150> US 62/476,541
   <151> 2017-03-24
<160> 14
<170> PatentIn version 3.5
<210> 1
   <211> 66
   <212> DNA
   <213> Artificial Sequence
<220>
   <223> Synthetic: N701 (forward adapter sequence)
<400> 1
   caagcagaag acggcatacg agattcgcct tagtctcgtg ggctcggaga tgtgtataag 60
   agacag 66
<210> 2
   <211> 68
   <212> DNA
   <213> Artificial Sequence
<220>
   <223> Synthetic: N701_rc (full length reverse complement)
<400> 2
   ctgtctctta tacacatctc cgagcccacg agactaaggc gaatctcgta tgccgtcttc 60
   tgcttgtt 68
<210> 3
   <211> 34
   <212> **DNA**
   <213> Artificial Sequence
<220>
   <223> Synthetic: N701_rc_34 (unprotected 3 end)
<400> 3
   ctgtctctta tacacatctc cgagcccacg agac 34
<210> 4
   <211> 34
   <212> DNA
   <213> Artificial Sequence
<220>
   <223> Synthetic: N701_rc_34Am (Amino modified 3 end)
<400> 4
   ctgtctctta tacacatctc cgagcccacg agac 34
<210> 5
   <211> 33
   <212> DNA
   <213> Artificial Sequence
<220>
   <223> Synthetic: N701_rc_34PT (Phosphorothioate modified 3 end)
<400> 5
   ctgtctctta tacacatctg acgctgccga cga 33
<210> 6
   <211> 70
   <212> DNA
   <213> Artificial Sequence
<220>
   <223> Synthetic: S502 (forward adapter sequence)
<400> 6
   aatgatacgg cgaccaccga gatctacacc tctctattcg tcggcagcgt cagatgtgta 60
   taagagacag 70
<210> 7
   <211> 72
   <212> DNA
   <213> Artificial Sequence
<220>
   <223> Synthetic: S502_rc (full length reverse complement)
<400> 7
   ctgtctctta tacacatctg acgctgccga cgaatagaga ggtgtagatc tcggtggtcg 60
   ccgtatcatt tt 72
<210> 8
   <211> 33
   <212> DNA
   <213> Artificial Sequence
<220>
   <223> Synthetic: S502_rc_33 (unprotected 3 end)
<400> 8
   ctgtctctta tacacatctg acgctgccga cga 33
<210> 9
   <211> 33
   <212> DNA
   <213> Artificial Sequence
<220>
   <223> Synthetic: S502_rc_33Am (Amino modified 3 end)
<400> 9
   ctgtctctta tacacatctg acgctgccga cga 33
<210> 10
   <211> 33
   <212> DNA
   <213> Artificial Sequence
<220>
   <223> Synthetic: S502_rc_33PT (Phosphorothioate modified 3 end)
<400> 10
   ctgtctctta tacacatctg acgctgccga cga 33
<210> 11
   <211> 33
   <212> DNA
   <213> Artificial Sequence
<220>
   <223> Synthetic: Read 1
<400> 11
   tcgtcggcag cgtcagatgt gtataagaga cag 33
<210> 12
   <211> 39
   <212> DNA
   <213> Artificial Sequence
<220>
   <223> Synthetic: Index 1 Read
<220>
   <221> misc_feature
   <222> (24)..(25)
   <223> i7 index site
<400> 12
   caagcagaag acggcatacg agatgtctcg tgggctcgg 39
<210> 13
   <211> 34
   <212> DNA
   <213> Artificial Sequence
<220>
   <223> Synthetic: Read 2L
<400> 13
   gtctcgtggg ctcggagatg tgtataagag acag 34
<210> 14
   <211> 43
   <212> DNA
   <213> Artificial Sequence
<220>
   <223> Synthetic: Index 2 Read
<220>
   <221> misc_feature
   <222> (29)..(30)
   <223> i5 index site
<400> 14
   aatgatacgg cgaccaccga gatctacact cgtcggcagc gtc 43